# EUROPÄISCHE PATENTSCHRIFT

(11) **EP 4 076 276 B1**
(45) Veröffentlichungstag und Bekanntmachung des Hinweises auf die Patenterteilung: **18.10.2023**
(21) Anmeldenummer: 19832380.0
(22) Anmeldetag: 20.12.2019
(51) Int. Cl.: A61F 2/16

(54) **INTRAOKULARLINSE**
INTRAOCULAR LENS
CRISTALLIN ARTIFICIEL

(43) Veröffentlichungstag der Anmeldung: 26.10.2022
(73) Patentinhaber: Carl Zeiss Meditec AG, 07745 Jena (DE)
(72) Erfinder: PANKIN, Dmitry, 12623 Berlin (DE)
(74) Vertreter: Carl Zeiss AG - Patentabteilung
(86) Internationale Anmeldenummer: PCT/EP2019/086603
(87) Internationale Veröffentlichungsnummer: WO 2021/121619

(56) Entgegenhaltungen:
- FR-A1- 2 749 161
- US-A- 6 129 760
- US-A1- 2005 107 875
- US-B2- 10 219 893

## Beschreibung

Die Erfindung betrifft eine Intraokularlinse, welche einen Optikkörper mit einer optischen Achse und einen an den Optikkörper angrenzenden plattenartigen Haptikkörper aufweist.

Eine Intraokularlinse ist als Ersatz für eine natürliche Augenlinse vorgesehen, wenn die natürliche Augenlinse durch Trübung oder Verhärtung nicht mehr ein gutes Sehen ermöglicht. Jede Intraokularlinse weist einen Optikkörper auf, durch den Lichtstrahlen bis zu einer Retina eines Auges eines Patienten gelangen können. Damit sich der Optikkörper möglichst mittig in einem Kapselsack des Auges des Patienten anordnen kann, grenzen an den Optikkörper Abstandshalter an, welche in einen Kontakt mit dem umgebenden Kapselsack kommen. Die Abstandshalter, die auch als Haptikkörper bezeichnet werden, sind oftmals C-förmig oder J-förmig ausgebildet. Solche Haptikkörper sind sehr schlank, sodass es möglich ist, die Intraokularlinse sehr stark zu falten und durch eine sehr kleine Inzision in den Kapselsack einzuführen.

Es kann jedoch vorteilhaft sein, dass eine Intraokularlinse mit einem Haptikkörper versehen ist, der plattenartig gestaltet ist. Eine solche Intraokularlinse lässt sich unmittelbar nach dem Implantieren in einen Kapselsack vom Operateur gut handhaben und mittig im Kapselsack zentrieren, wobei nur eine geringe Gefahr besteht, bei der Handhabung des Haptikkörpers den Optikkörper zu beschädigen. Da ein plattenartiger Haptikkörper eine relativ große Außenkante aufweist, ist auch eine relativ große Kontaktfläche zur Innenwand eines Kapselsackes erreichbar. Dies bedeutet eine auch auf lange Zeit betrachtet stabile Lage der Intraokularlinse im Kapselsack.

Nachteilig ist jedoch, dass bei einem relativ kleinen Kapselsack eine Intraokularlinse mit einem plattenartigen Haptikkörper unter großer mechanischer Spannung steht. Dies kann dazu führen, dass sich eine solche Intraokularlinse entlang ihrer Längsachse durchbiegt. Die Folge kann ein axialer Versatz des Optikkörpers in dem Kapselsack sein, wodurch sich der Brennpunkt der Intraokularlinse vor oder hinter die Netzhaut verlagert, was mit einem Verlust an Schärfe einhergeht. Wenn die Intraokularlinse an ihrer Rückseite mit einer PCI-Kante zur Verhinderung von Nachstar ausgestattet ist, kann es passieren, dass die Intraokularlinse nach einem solchen axialen Versatz nicht mehr wie vorgesehen an der Rückwand des Kapselsackes anliegt. Dadurch können die Zellen, die einen Nachstar bilden, in den zentrischen Bereich der Rückwand des Kapselsackes vordringen und eine erneute Trübung der Sicht bewirken. Eine in einem zu kleinen Kapselsack eingesetzte Intraokularlinse mit plattenartiger Haptik kann sich dabei auch derart verformen, dass der Optikkörper eine astigmatische Verzerrung erfährt.

Intraokularlinsen mit einem Haptikkörper sind zum Beispiel in WO 01/ 89 425 A1 und WO 2006/ 033 984 A1 beschrieben.

Eine Intraokularlinse nach dem Oberbegriff des Anspruchs 1 ist aus dem Dokument US-A- 6 129 760 bekannt.

Es ist eine Aufgabe der Erfindung, eine Intraokularlinse mit plattenartigem Haptikkörper zu schaffen, die auch bei einem relativ kleinen Kapselsack ein gutes Sehen ermöglicht.

Die Aufgabe wird durch den Gegenstand des Anspruchs 1 gelöst. Vorteilhafte Weiterbildungen der Erfindung sind Gegenstand der Unteransprüche.

Die erfindungsgemäße Intraokularlinse weist auf:
- einen Optikkörper mit einem geometrischen Mittelpunkt, durch welchen eine erste Hauptachse als Längsachse und eine zweite Hauptachse als Querachse verläuft, wobei die Querachse senkrecht zur Längsachse der Intraokularlinse angeordnet ist,
- einen an den Optikkörper angrenzenden plattenartigen ersten Haptikkörper und einen an den Optikkörper angrenzenden plattenartigen zweiten Haptikkörper, wobei der erste Haptikkörper und der zweite Haptikkörper punktsymmetrisch zur optischen Achse angeordnet sind,

wobei ein Außenradius der Intraokularlinse um den geometrischen Mittelpunkt und ein radialer Abstand von dem geometrischen Mittelpunkt zu einem Schnittpunkt der Querachse mit einer Umfangslinie der Intraokularlinse ein Verhältnis zueinander aufweisen, welches im Bereich von 1:0,5 bis 1:0,9 liegt,
wobei in dem ersten Haptikkörper mindestens zwei erste Aussparungen vorhanden sind,
wobei die eine erste Aussparung linksseitig von der Längsachse und die weitere erste Aussparung rechtseitig von der Längsachse vorhanden sind, wobei die zwei ersten Aussparungen eine jeweilige erste Aussparungslänge und erste Aussparungsbreite aufweisen,
wobei die erste Aussparungslänge größer als die erste Aussparungsbreite ist,

wobei die ersten Aussparungen jeweils einen Flächenschwerpunkt aufweisen, wobei sich diese jeweiligen Flächenschwerpunkte mit einer Linie verbinden lassen, welche einen ersten Kreisbogen mit einem ersten Radius um einen ersten Mittelpunkt bildet, der auf der Längsachse angeordnet ist,
wobei in dem ersten Haptikkörper mindestens zwei zweite Aussparungen vorhanden sind, wobei die eine zweite Aussparung linksseitig von der Längsachse und die weitere zweite Aussparung rechtseitig von der Längsachse vorhanden sind, wobei die zwei zweiten Aussparungen eine jeweilige zweite Aussparungslänge und zweite Aussparungsbreite aufweisen, wobei die zweite Aussparungslänge größer als die zweite Aussparungsbreite ist,
wobei die zweiten Aussparungen jeweils einen Flächenschwerpunkt aufweisen, wobei sich diese jeweiligen Flächenschwerpunkte mit einer Linie verbinden lassen, welche einen zweiten Kreisbogen mit einem zweiten Radius um den ersten Mittelpunkt bildet, wobei der zweite Radius kleiner als der erste Radius ist.

Die Intraokularlinse weist somit einen Optikkörper auf, der in der Draufsicht betrachtet einen geometrischen Mittelpunkt besitzt. Meistens ist der geometrische Mittelpunkt der Intraokularlinse ein Punkt, der von einer optischen Achse des Optikkörpers durchdrungen wird. In einer Draufsicht der Intraokularlinse lassen sich eine erste Hauptachse der Intraokularlinse und eine zweite Hauptachse der Intraokularlinse angeben. Die erste Hauptachse ist eine Längsachse, welche sich in Richtung der größten Ausdehnung bzw. in der Längsrichtung der Intraokularlinse erstreckt. Bei der erfindungsgemäßen Intraokularlinse verläuft ebenfalls in der Draufsicht senkrecht zu der ersten Hauptachse eine zweite Hauptachse, welche eine Querachse der Intraokularlinse darstellt.

An den Optikkörper grenzen ein plattenartiger erster Haptikkörper und ein plattenartiger zweiter Haptikkörper an. Beide Haptikkörper sind punktsymmetrisch zum geometrischen Mittelpunkt der Intraokularlinse angeordnet. Bevorzugt erstrecken sich der erste Haptikkörper und der zweite Haptikkörper entlang der Längsachse.

Um die Intraokularlinse kann in der Draufsicht ein Umkreis mit einem Außenradius um den geometrischen Mittelpunkt herum gelegt werden. Der Außenradius kann in ein Verhältnis gesetzt werden mit einem radialen Abstand, der von dem geometrischen Mittelpunkt zu einem Schnittpunkt der Querachse mit einer Umfangslinie der Intraokularlinse gebildet ist. Bei der erfindungsgemäßen Intraokularlinse ist dieses Verhältnis aus dem Außenradius mit dem radialen Abstand in einem Bereich von 1:0,5 bis 1:0,9, bevorzugt im Bereich von 1:0,5 bis 1:0,8, oder bevorzugt im Bereich von 1:0,5 bis 1:0,7, oder bevorzugt im Bereich von 1:0,5 bis 1:0,6. Die Intraokularlinse erstreckt sich somit mehr entlang der Längsachse als entlang der Querachse.

Die Intraokularlinse weist in dem ersten Haptikkörper mindestens zwei erste Aussparungen auf. Die eine erste Aussparung ist linksseitig von der Längsachse und die weitere erste Aussparung ist rechtsseitig von der Längsachse angeordnet. Ferner weist die Intraokularlinse in dem ersten Haptikkörper mindestens zwei zweite Aussparungen auf, wobei die eine zweite Aussparung linksseitig von der Längsachse und die weitere zweite Aussparung rechtsseitig von der Längsachse angeordnet sind. Bevorzugt weist die Intraokularlinse in dem ersten Haptikkörper noch mindestens eine dritte Aussparung auf, wobei diese dritte Aussparung die Längsachse schneidet. Die dritte Aussparung weist eine dritte Aussparungslänge und eine dritte Aussparungsbreite auf, wobei die dritte Aussparungslänge größer als die dritte Aussparungsbreite ist.

Bevorzugt weist die Intraokularlinse im zweiten Haptikkörper mindestens zwei erste Aussparungen auf. Die eine erste Aussparung ist linksseitig von der Längsachse und die weitere erste Aussparung ist rechtsseitig von der Längsachse angeordnet. Ferner weist die Intraokularlinse in dem zweiten Haptikkörper mindestens zwei zweite Aussparungen auf, wobei die eine zweite Aussparung linksseitig von der Längsachse und die weitere zweite Aussparung rechtsseitig von der Längsachse angeordnet sind. Bevorzugt weist die Intraokularlinse in dem zweiten Haptikkörper noch mindestens eine dritte Aussparung auf, wobei diese dritte Aussparung die Längsachse schneidet. Die dritte Aussparung weist eine dritte Aussparungslänge und eine dritte Aussparungsbreite auf, wobei die dritte Aussparungslänge größer als die dritte Aussparungsbreite ist.

Die Aussparungen bewirken, dass bei einer Druckkraft, welche in Richtung der Längsachse und parallel zur Längsachse der Intraokularlinse gerichtet ist, also vom ersten Haptikkörper oder zweiten Haptikkörper in Richtung zum Optikkörper hin, eine elastische Verformung des ersten Haptikkörpers oder zweiten Haptikkörpers in Richtung zum Optikkörper erfolgt. Eine Aussparung kann in einem Ersatzschaltbild als elastische Feder betrachtet werden. Da nicht nur erste Aussparungen, sondern auch zweite Aussparungen vorgesehen sind, welche sich jeweils verformen können, wobei die ersten Aussparungen auf einem Kreisbogen mit einem ersten Radius um einen Mittelpunkt und die zweiten Aussparungen auf einem Kreisbogen mit einem zweiten Radius um den Mittelpunkt, der kleiner als der erste Radius ist, angeordnet sind, bedeutet dies bei einer Druckkraft vom ersten Haptikkörper oder zweiten Haptikkörper in Richtung zum Optikkörper eine Reihenschaltung von elastischen Federn. Der gesamte Federweg teilt sich damit auf die ersten Aussparungen und zweiten Aussparungen auf, sodass jede Aussparung nur einen relativ kurzen Federweg erfährt. Die Haptik kann damit bei einem relativ kleinen Kapselsack in ihrer Länge, also entlang ihrer Längsachse, deutlich verkürzt werden.

Da von einer ersten Aussparung oder einer zweiten Aussparung die jeweilige Aussparungslänge größer als die jeweilige Aussparungsbreite ist, besitzt der Haptikkörper trotzdem eine gute Torsionssteifigkeit, sodass bei einer Druckkraft, welche auf den Haptikkörper in Richtung der Längsachse und parallel zur Längsachse gerichtet ist, der Optikkörper nicht verformt wird und keine astigmatische Verzerrung des Optikkörpers auftritt. Da keine Verformung des Optikkörpers auftritt, ist auch eine gute Anlage des Optikkörpers an die innere Rückwand des Kapselsackes sichergestellt, sodass eine PCI-Kante an der Intraokularlinse unverändert gut wirken kann und eine Nachstar-Bildung vermieden wird.

Bevorzugt ist die erste Aussparung oder die zweite Aussparung schlitzförmig ausgebildet. Damit kann ein Verhältnis von Aussparungslänge zu Aussparungsbreite von deutlich größer als 1 erreicht werden, sodass eine große Elastizität in der Längsachse der Intraokularlinse bei gleichzeitig hoher Torsionssteifigkeit erreicht wird. Druckkräfte an einer Umfangswand des Haptikkörpers der Intraokularlinse können gleichmäßig aufgenommen werden. Zudem ist die Herstellung eines Schlitzes in mechanischer Hinsicht relativ einfach.

Es ist vorteilhaft, wenn die Intraokularlinse aus einem einzigen Werkstoff gebildet ist. Damit müssen keine zusätzlichen Elemente wie z.B. Drähte für eine höhere Steifigkeit in einer Dimension integriert werden. Eine Intraokularlinse aus einem einzigen Werkstoff ermöglicht zudem mit großer Genauigkeit homogene mechanische Eigenschaften. Damit ist eine starke Faltung mit einem Injektor für eine Mikroinzision gut möglich, wobei ein gleichmäßiges Aufklappen im Kapselsack in relativ kurzer Zeit erreichbar ist.

Bevorzugt sind die erste Aussparung oder zweite Aussparung nur innerhalb des ersten Haptikkörpers und/oder nur innerhalb des zweiten Haptikkörpers vorhanden und erreichen einen Außenrand des ersten Haptikkörpers und/oder zweiten Haptikkörpers nicht. Damit kann zusätzlich eine höhere Torsionssteifigkeit der Intraokularlinse um ihre Längsachse sichergestellt werden.

Die erste Aussparung und/oder zweite Aussparung können jeweils eine Aussparungsbreite in einem Bereich von 0,05 mm bis 0,8 mm, bevorzugt von 0,05 mm bis 0,4 mm, und besonders bevorzugt von 0,1 mm bis 0,3 mm aufweisen. Damit lässt sich eine hohe Elastizität des Haptikkörpers in der Längsachse der Intraokularlinse bei gleichzeitig hoher Torsionssteifigkeit erreichen.

In einer weiteren Ausführungsform weist die Intraokularlinse eine Oberseite und eine Unterseite auf und die erste Aussparung oder zweite Aussparung reicht im Längsquerschnitt von der Oberseite bis zur Unterseite. Eine Aussparung ist somit ein Durchbruch oder ein Loch, sodass eine sehr hohe Elastizität des Haptikkörpers in der Längsachse der Intraokularlinse bei gleichzeitig hoher Torsionssteifigkeit möglich ist.

Bevorzugt weist die erste Aussparung oder zweite Aussparung im Längsquerschnitt an der Oberseite der Intraokularlinse eine größere Breite als an der Unterseite der Intraokularlinse auf. Damit ist die Biegesteifigkeit an der Oberseite geringer als an der Unterseite, sodass eine Durchbiegung entlang der Längsachse in eine definierte Richtung, also zur Oberseite hin, erfolgen kann. Damit wird eine höhere Sicherheit erreicht, dass die Rückwand des Optikkörpers an der Innenseite der Kapselwand zuverlässig anliegt und kein axialer Versatz der Intraokularlinse entlang der optischen Achse auftritt.

Weitere Vorteile und Merkmale der Erfindung werden mit Bezug auf die nachfolgenden Zeichnungen erklärt, in welchen zeigen:
- Fig. 1: eine schematische Darstellung einer Intraokularlinse gemäß einer ersten Ausführungsform in einer Draufsicht;
- Fig. 2: eine schematische Darstellung einer Intraokularlinse gemäß einer zweiten Ausführungsform in einer Draufsicht;
- Fig. 3: eine Intraokularlinse gemäß einer dritten Ausführungsform in einer perspektivischen Ansicht;
- Fig. 4a: eine schematische Darstellung eines Teils einer Intraokularlinse gemäß der dritten Ausführungsform in einer Draufsicht in einem entspannten Zustand;
- Fig. 4b: eine schematische Darstellung eines Teils einer Intraokularlinse gemäß der dritten Ausführungsform in einer Draufsicht in einem mit einer Druckkraft beaufschlagten Zustand;
- Fig. 5a: eine schematische Darstellung eines Teils einer Intraokularlinse gemäß einer vierten Ausführungsform in einem Längsquerschnitt in einem entspannten Zustand;
- Fig. 5b: eine schematische Darstellung eines Teils einer Intraokularlinse gemäß der vierten Ausführungsform in einem Längsquerschnitt in einem mit einer Druckkraft beaufschlagten Zustand; und
- Fig. 6: eine schematische Darstellung eines Teils eines Haptikkörpers einer Intraokularlinse gemäß einer fünften Ausführungsform in einem Längsquerschnitt.

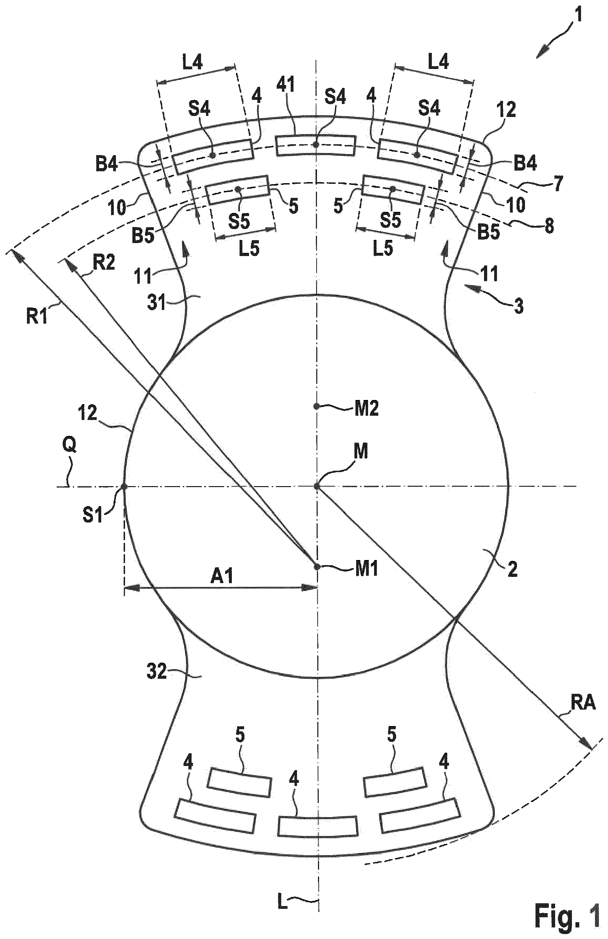

Fig. 1 zeigt eine Intraokularlinse 1 mit einem Optikkörper 2 mit einer optischen Achse A, welche senkrecht zur Zeichenebene und senkrecht zu einer Längsachse L der Intraokularlinse 1 verläuft. An den Optikkörper 2 grenzt ein plattenartiger Haptikkörper 3 an, der einstückig mit dem Optikkörper 2 ausgebildet ist. Der Haptikkörper 3 weist einen ersten Haptikkörper 31 und einen zweiten Haptikkörper 32 auf, wobei der erste Haptikkörper 31 und der zweite Haptikkörper 32 punktsymmetrisch zur geometrischen Mitte M angeordnet sind.

Wird um den geometrischen Mittelpunkt M ein Umkreis um die Intraokularlinse 1 gebildet, so besitzt dieser Umkreis einen Außenradius RA. Ein radialer Abstand A1 ist derart definiert, dass er den Abstand zwischen dem geometrischen Mittelpunkt M und einem Schnittpunkt S1 der Querachse Q mit einer Umfangslinie 12 der Intraokularlinse 1 bildet. Von Bedeutung ist das Verhältnis aus Außenradius RA zu radialem Abstand A1, welches im Bereich von 1:0,5 bis 1:0,9 liegt. Damit ist eine Intraokularlinse 1 beschrieben, welche eine größere Erstreckung entlang der Längsachse L aufweist als entlang der Querachse Q. Bei einer solchen Intraokularlinse besteht das Problem, dass sie sich in einem relativ kleinen Kapselsack derart verformen kann, dass sie sich entlang ihrer Längsachse durchbiegt. Dies kann zu einem axialen Versatz des Optikkörpers im Kapselsack oder zu einer astigmatischen Verzerrung des Optikkörpers führen.

Um diese Folgen zu vermeiden, weist die erfindungsgemäße Intraokularlinse in dem ersten Haptikkörper 31 eine erste Aussparung 4 linksseitig von der Längsachse L und eine weitere erste Aussparung 4 rechtsseitig von der Längsachse L auf. Die beiden ersten Aussparungen 4 sind symmetrisch zur Längsachse L angeordnet. Zusätzlich weist der erste Haptikkörper 31 eine zweite Aussparung 5 linksseitig von der Längsachse L und eine weitere zweite Aussparung 5 rechtsseitig von der Längsachse L auf. Die beiden zweiten Aussparungen 5 sind symmetrisch zur Längsachse L angeordnet.

Die erste Aussparung 4 weist eine erste Aussparungslänge L4 und eine erste Aussparungsbreite B4 auf, wobei die erste Aussparungslänge L4 größer als die erste Aussparungsbreite B4 ist. Jede der ersten Aussparungen 4 besitzt einen ersten Flächenschwerpunkt S4. Die erfindungsgemäße Intraokularlinse 1 ist so ausgebildet, dass sich die Flächenschwerpunkte S4 der ersten Aussparungen 4 mit einer Linie verbinden lassen, welche einen ersten Kreisbogen 7 mit einem ersten Radius R1 um einen Mittelpunkt M1 bildet. Der Mittelpunkt M1 ist auf der Längsachse L angeordnet. In einer bevorzugten Ausführungsform liegt der Mittelpunkt M1 an der gleichen Position wie der geometrische Mittelpunkt M der Intraokularlinse 1.

Die zweite Aussparung 5 weist eine zweite Aussparungslänge L5 und eine zweite Aussparungsbreite B5 auf, wobei die zweite Aussparungslänge L5 größer als die zweite Aussparungsbreite B5 ist. Jede der zweiten Aussparungen 5 besitzt einen zweiten Flächenschwerpunkt S5, wobei sich diese Flächenschwerpunkte S5 mit einer Linie verbinden lassen, die einen zweiten Kreisbogen 8 mit einem zweiten Radius R2 um den Mittelpunkt M1 bildet. Der zweite Radius R2 ist kleiner als der erste Radius R1, sodass die zweiten Aussparungen 5 näher zum Optikkörper 2 angeordnet sind als die ersten Aussparungen 4.

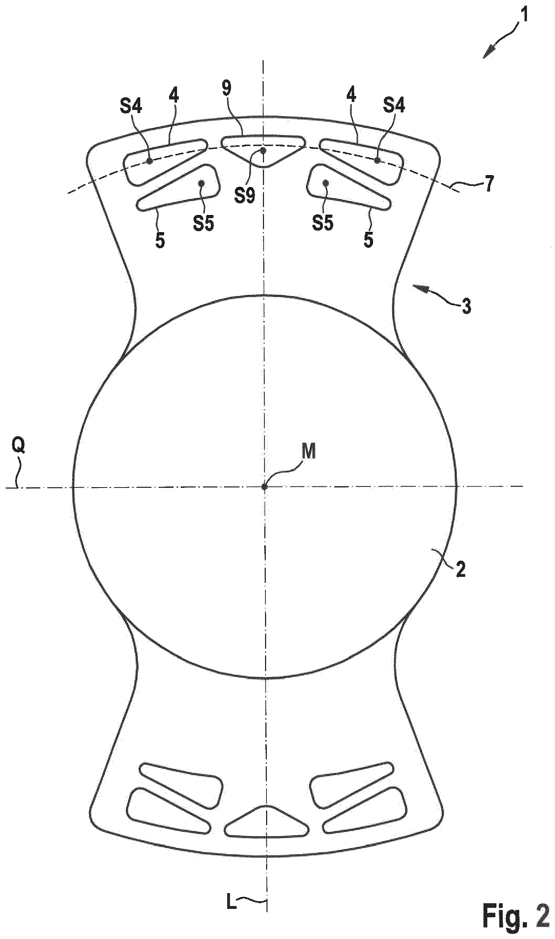

Die ersten Aussparungen 4 und die zweiten Aussparungen 5 sind bei dieser ersten Ausführungsform schlitzartig ausgebildet. Sie können jedoch auch mit anderen Geometrien, zum Beispiel dreiecksförmig oder tropfenförmig, gestaltet sein, siehe Fig. 2. Es kann zwischen oder neben den beiden ersten Aussparungen 4 auch mindestens eine dritte Aussparung 41 vorgesehen sein, deren Flächenschwerpunkt S4 ebenfalls auf dem Kreisbogen 7 liegt, siehe Fig. 1, wobei diese dritte Aussparung die Längsachse L schneidet. Die dritte Aussparung 41 weist eine dritte Aussparungslänge und eine dritte Aussparungsbreite auf, wobei die dritte Aussparungslänge größer als die dritte Aussparungsbreite ist. Es kann auch vorgesehen sein, dass zwischen den beiden ersten Aussparungen 4 eine zusätzliche Aussparung 9 mit einer anderen Geometrie als die ersten Aussparungen 4 vorgesehen ist, siehe Fig. 2, wobei es möglich ist, dass sich deren Flächenschwerpunkt S9 nicht auf dem Kreisbogen 7 befindet.

Bei der in Fig. 1 und Fig. 2 dargestellten Intraokularlinse 1 sind die Aussparungen 4, 5 im ersten Haptikkörper 31 und im zweiten Haptikkörper 32 vorgesehen. Bei den Aussparungen 4, 5, die sich im zweiten Haptikkörper 32 befinden, lassen sich die jeweiligen Flächenschwerpunkte der Aussparungen 4, 5 ebenfalls mit einer Linie verbinden, welche einen Kreisbogen mit einem Radius um einen Mittelpunkt bildet. Für den zweiten Haptikkörper 32 ist bei der in Fig. 1 gezeigten Ausführungsform der zugehörige Mittelpunkt der Mittelpunkt M2.

Bei der in Fig. 1 gezeigten Intraokularlinse 1 sind die zweiten Aussparungen 5 genauso wie die ersten Aussparungen 4 schlitzförmig ausgebildet. Es ist möglich, dass zwischen den zweiten Aussparungen 5 mindestens eine zusätzliche zweite Aussparung 5 vorgesehen ist.

Vorteilhaft ist es, wenn die Aussparungen 4, 5 nur innerhalb des ersten Haptikkörpers 31 oder zweiten Haptikkörpers 32 vorgesehen sind und einen Außenrand 10 des ersten Haptikkörpers 31 oder zweiten Haptikkörpers 32 nicht erreichen. Damit bleibt eine Randzone 11 am ersten Haptikkörper 31 oder zweiten Haptikkörper 32 zwischen den Aussparungen 4, 5 und dem Außenrand 10 des ersten Haptikkörpers 31 oder zweiten Haptikkörpers 32 bestehen, sodass eine relativ hohe Torsionssteifigkeit des ersten Haptikkörpers 31 oder zweiten Haptikkörpers 32 um die Längsachse L erreichbar ist. Eine Breite der Randzone 11 und somit ein Abstand zwischen den Aussparungen 4, 5 und dem Außenrand 10 des ersten Haptikkörpers 31 oder zweiten Haptikkörpers 32 ist bevorzugt der Betrag von der Breite B4 der ersten Aussparung 4 oder der Breite B5 der zweiten Aussparung 5. Besonders bevorzugt erreicht die Breite der Randzone 11, also der Abstand zwischen den Aussparungen 4, 5 und dem Außenrand 10 des ersten Haptikkörpers 31 oder zweiten Haptikkörpers 32, einen Betrag im Bereich von einmal bis zweimal der Breite B4 der ersten Aussparung 4 oder im Bereich von einmal bis zweimal der Breite B5 der zweiten Aussparung 5.

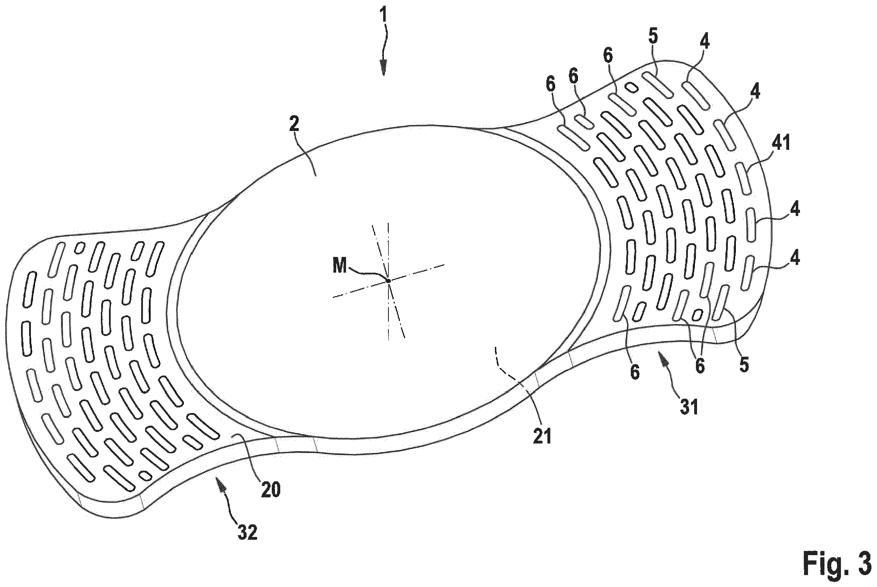

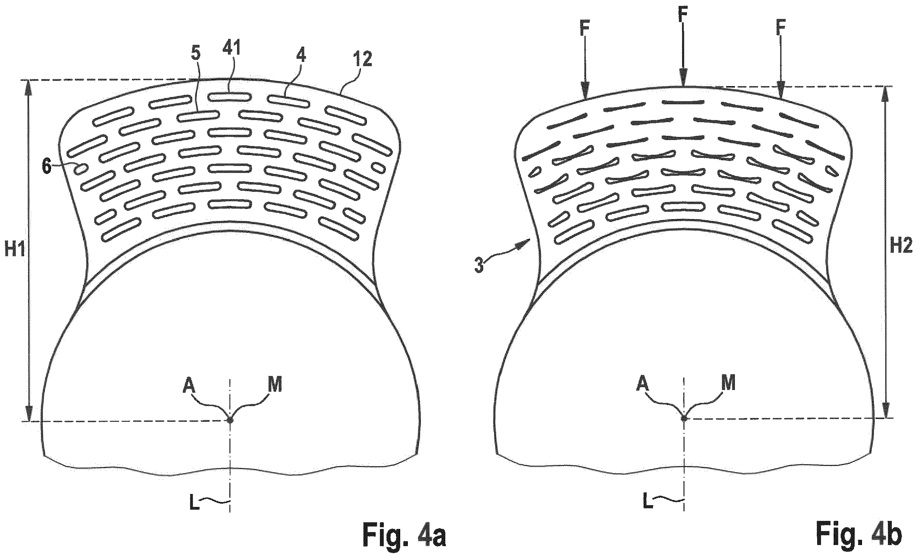

Gemäß einer dritten Ausführungsform können noch weitere Aussparungen 6 vorgesehen sein, deren jeweilige Flächenschwerpunkte sich mit einer Linie verbinden lassen, die einen weiteren Kreisbogen mit einem dritten Radius um den Mittelpunkt M1 bildet, wobei der weitere Radius kleiner als der zweite Radius R2 ist, siehe Fig. 3 und Fig. 4. Die weiteren Aussparungen 6 können bis zum Optikkörper 2 heranreichen, so dass der Haptikkörper 3 eine Vielzahl an Aussparungen 4, 5, 6, 9, 41 aufweist.

Aus Fig. 3 ist erkennbar, dass die Intraokularlinse 1 eine Oberseite 20 und eine Unterseite 21 aufweist. Bevorzugt reichen die ersten Aussparungen 4 und die zweiten Aussparungen 5 im Längsquerschnitt der Intraokularlinse 1 von der Oberseite 20 zur Unterseite 21. Eine solche Aussparung 4, 5 ist somit ein Durchbruch oder ein Langloch. Eine solche Ausführungsform ist aus folgendem Grund besonders vorteilhaft:
Wenn eine Intraokularlinse 1 in einem Kapselsack eingesetzt ist und dieser auf eine Umfangslinie 12 der Intraokularlinse 1 am Rand des Haptikkörpers 3 eine relativ starke Druckkraft F in Richtung und parallel zur Längsachse L ausübt, weil die Intraokularlinse 1 in einem entspannten Zustand, siehe Fig. 4a, für diesen Kapselsack zu groß ist, führt dies zu einer relativ großen elastischen Verformung der Aussparungen 4, 41, 5, und 6 im Haptikkörper 3, siehe Fig. 4b. Im entspannten Zustand der Intraokularlinse 1 liegt von der geometrischen Mitte M, durch welche bei dieser Ausführungsform die optische Achse A der Intraokularlinse 1 verläuft, bis zum Schnittpunkt der Umfangslinie 12 mit der Längsachse L eine Höhe H1 vor. Wenn eine Druckkraft auf die Umfangslinie 12 einwirkt, verringert sich die Höhe zu einer Höhe H2. Aussparungen 4, 41, 5 und 6, die von der Oberseite 20 bis zur Unterseite 21 reichen, ermöglichen somit eine relativ große Kompression ähnlich einem Schwamm.

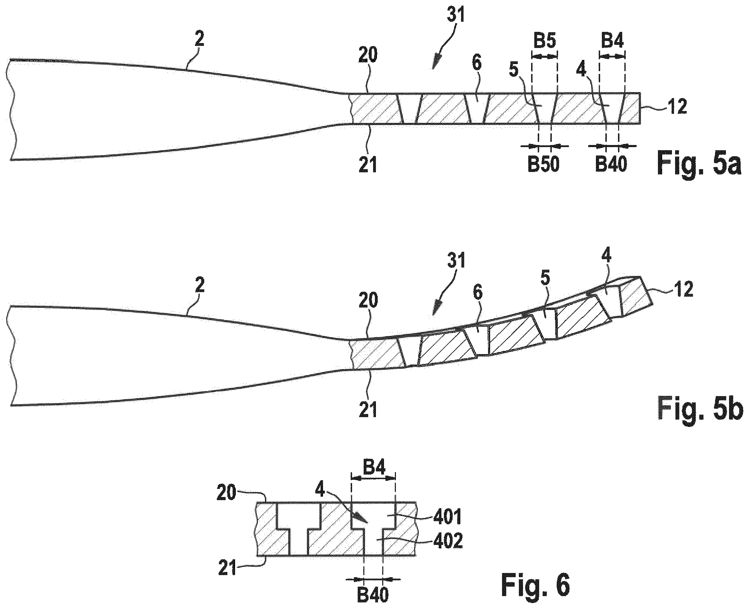

Dieser Effekt kann noch verstärkt werden, wenn die erste Aussparung 4 im Längsquerschnitt an der Oberseite 20 eine Breite B4 und an der Unterseite 21 eine Breite B40 aufweist, wobei die Breite B4 an der Oberseite 20 größer ist als die Breite B40 an der Unterseite. Fig. 5a zeigt eine solche Intraokularlinse 1 im entspannten Zustand. Wenn am oberen Rand auf die Umfangslinie 12 des ersten Haptikkörpers 31 eine Druckkraft in Richtung und parallel zu der Längsachse L einwirkt, biegt sich der erste Haptikkörper 31 zu der Seite hin, an der die Aussparung 4 die größere Breite B4 besitzt, also zur Oberseite 20 hin, siehe Fig. 5b. Damit bleibt der Optikkörper 2 unverändert in seiner Lage und erfährt keinen axialen Versatz entlang der optischen Achse A. Die Änderung der Breite von B4 zu B40 kann entweder stetig oder gestuft erfolgen, siehe Fig. 6. Wenn dies gestuft erfolgt, weist die erste Aussparung ein Sackloch 401 mit einer Breite B4 auf, welches von der Oberseite 20 nicht bis zur Unterseite 21 reicht, wobei dieses Sackloch 401 von einem anderen Sackloch 402 fortgesetzt wird, welches eine konstante Breite B40 besitzt.

Diese Ausführungen gelten analog auch zu einer zweiten Aussparung 5 mit einer Breite B5 an der Oberseite und einer Breite B50 an der Unterseite. Entsprechendes gilt auch für die weiteren Aussparungen 6.

## Patentansprüche

1. Intraokularlinse (1), welche aufweist:
- einen Optikkörper (2) mit einem geometrischen Mittelpunkt (M), durch welchen eine erste Hauptachse als Längsachse (L) und eine zweite Hauptachse als Querachse (Q) verläuft, wobei die Querachse (Q) senkrecht zur Längsachse (L) der Intraokularlinse (1) angeordnet ist,
- einen an den Optikkörper (2) angrenzenden plattenartigen ersten Haptikkörper (31) und einen an den Optikkörper (2) angrenzenden plattenartigen zweiten Haptikkörper (32), **dadurch gekennzeichnet, dass**
der erste Haptikkörper (31) und der zweite Haptikkörper (32) punktsymmetrisch zum geometrischen Mittelpunkt (M) angeordnet sind,
wobei ein Außenradius (RA) der Intraokularlinse (1) um den geometrischen Mittelpunkt M und ein radialer Abstand (A1) von dem geometrischen Mittelpunkt (M) zu einem Schnittpunkt (S1) der Querachse (Q) mit einer Umfangslinie (12) der Intraokularlinse (1) ein Verhältnis zueinander aufweisen, welches im Bereich von 1:0,5 bis 1:0,9 liegt,
wobei in dem ersten Haptikkörper (31) mindestens zwei erste Aussparungen (4) vorhanden sind, wobei die eine erste Aussparung (4) linksseitig von der Längsachse (L) und die weitere erste Aussparung (4) rechtseitig von der Längsachse (L) vorhanden sind, wobei die zwei ersten Aussparungen (4) eine jeweilige erste Aussparungslänge (L4) und eine erste Aussparungsbreite (B4) aufweisen, wobei die erste Aussparungslänge (L4) größer als die erste Aussparungsbreite (B4) ist,
wobei die ersten Aussparungen (4) jeweils einen Flächenschwerpunkt (S4) aufweisen, wobei sich diese jeweiligen Flächenschwerpunkte (S4) mit einer Linie verbinden lassen, welche einen ersten Kreisbogen (7) mit einem ersten Radius (R1) um einen ersten Mittelpunkt (M1) bildet, der auf der Längsachse (L) angeordnet ist,
wobei in dem ersten Haptikkörper (31) mindestens zwei zweite Aussparungen (5) vorhanden sind, wobei die eine zweite Aussparung (5) linksseitig von der Längsachse (L) und die weitere zweite Aussparung (5) rechtseitig von der Längsachse (L) vorhanden sind, wobei die zwei zweiten Aussparungen (5) eine jeweilige zweite Aussparungslänge (L5) und eine zweite Aussparungsbreite (B5) aufweisen, wobei die zweite Aussparungslänge (L5) größer als die zweite Aussparungsbreite (B5) ist,
wobei die zweiten Aussparungen (5) jeweils einen Flächenschwerpunkt (S5) aufweisen, wobei sich diese jeweiligen Flächenschwerpunkte (S5) mit einer Linie verbinden lassen, welche einen zweiten Kreisbogen (8) mit einem zweiten Radius (R2) um den ersten Mittelpunkt (M1) bildet, wobei der zweite Radius (R2) kleiner als der erste Radius (R1) ist.

2. Intraokularlinse (1) nach Anspruch 1, wobei die erste Aussparung (4) oder zweite Aussparung (5) schlitzförmig ausgebildet ist.

3. Intraokularlinse (1) nach einem der vorherigen Ansprüche, wobei die Intraokularlinse (1) aus einem einzigen Werkstoff gebildet ist

4. Intraokularlinse (1) nach einem der vorherigen Ansprüche, wobei die erste Aussparung (4) oder zweite Aussparung (5) nur innerhalb des ersten Haptikkörpers (31) vorhanden sind und einen Außenrand (10) des Haptikkörpers (3) nicht erreichen.

5. Intraokularlinse (1) nach einem der vorherigen Ansprüche, wobei die erste Aussparung (4) eine erste Aussparungsbreite (B4) im Bereich von 0,05 mm bis 0,8 mm und/oder die zweite Aussparung (5) eine zweite Aussparungsbreite (B5) im Bereich von 0,05 mm bis 0,8 mm aufweisen.

6. Intraokularlinse (1) nach einem der vorherigen Ansprüche, wobei die Intraokularlinse (1) eine Oberseite (20) und eine Unterseite (21) aufweist und die erste Aussparung (4) oder zweite Aussparung (5) im Längsquerschnitt von der Oberseite (20) der Intraokularlinse (1) bis zur Unterseite (21) der Intraokularlinse (1) reicht.

7. Intraokularlinse (1) nach Anspruch 6, wobei die erste Aussparung (4) oder zweite Aussparung (5) im Längsquerschnitt an der Oberseite (20) der Intraokularlinse (1) eine größere Breite (B4) als an der Unterseite (21) der Intraokularlinse (1) aufweist.

## Claims

1. Intraocular lens (1), which has:
- an optic (2) with a geometric centre point (M), through which a first main axis extends as a longitudinal axis (L) and a second main axis extends as a transverse axis (Q), wherein the transverse axis (Q) is arranged perpendicular to the longitudinal axis (L) of the intraocular lens (1),
- a plate-like first haptic (31), adjacent to the optic (2), and a plate-like second haptic (32), adjacent to the optic (2),
**characterized in that** the first haptic (31) and the second haptic (32) are arranged point-symmetrically in relation to the geometric centre point (M), wherein an outer radius (RA) of the intraocular lens (1) about the geometric centre point M and a radial distance (A1) from the geometric centre point (M) to an intersection point (S1) of the transverse axis (Q) with a circumferential line (12) of the intraocular lens (1) have a ratio to one another which lies in the range from 1:0.5 to 1:0.9,
wherein in the first haptic (31) there are at least two first clearances (4), wherein the one first clearance (4) is on the left of the longitudinal axis (L) and the other first clearance (4) is on the right of the longitudinal axis (L), wherein the two first clearances (4) have a respective first clearance length (L4) and a first clearance width (B4), wherein the first clearance length (L4) is greater than the first clearance width (B4),
wherein the first clearances (4) respectively have a centroid (S4), wherein these respective centroids (S4) can be connected to a line which forms a first arc (7) with a first radius (R1) about a first centre point (M1), which is arranged on the longitudinal axis (L),
wherein in the first haptic (31) there are at least two second clearances (5), wherein the one second clearance (5) is on the left of the longitudinal axis (L) and the other second clearance (5) is on the right of the longitudinal axis (L), wherein the two second clearances (5) have a respective second clearance length (L5) and a second clearance width (B5), wherein the second clearance length (L5) is greater than the second clearance width (B5), wherein the second clearances (5) respectively have a centroid (S5), wherein these respective centroids (S5) can be connected to a line which forms a second arc (8) with a second radius (R2) about the first centre point (M1), wherein the second radius (R2) is smaller than the first radius (R1).

2. Intraocular lens (1) according to Claim 1, wherein the first clearance (4) or the second clearance (5) takes the form of a slit.

3. Intraocular lens (1) according to one of the preceding claims, wherein the intraocular lens (1) is formed from a single material.

4. Intraocular lens (1) according to one of the preceding claims, wherein the first clearance (4) or second clearance (5) are only within the first haptic (31) and do not reach an outer edge (10) of the haptic (3) .

5. Intraocular lens (1) according to one of the preceding claims, wherein the first clearance (4) has a first clearance width (B4) in the range from 0.05 mm to 0.8 mm and/or the second clearance (5) has a second clearance width (B5) in the range from 0.05 mm to 0.8 mm.

6. Intraocular lens (1) according to one of the preceding claims, wherein the intraocular lens (1) has an upper side (20) and an underside (21) and, in the longitudinal cross section, the first clearance (4) or second clearance (5) reaches from the upper side (20) of the intraocular lens (1) to the underside (21) of the intraocular lens (1).

7. Intraocular lens (1) according to Claim 6, wherein, in the longitudinal cross section, the first clearance (4) or second clearance (5) has a greater width (B4) on the upper side (20) of the intraocular lens (1) than on the underside (21) of the intraocular lens (1).

## Revendications

1. Lentille intraoculaire (1), qui présente :
- un corps optique (2) avec un centre géométrique (M), à travers lequel s'étendent un premier axe principal en tant qu'axe longitudinal (L) et un deuxième axe principal en tant qu'axe transversal (Q), l'axe transversal (Q) étant agencé perpendiculairement à l'axe longitudinal (L) de la lentille intraoculaire (1),
- un premier corps haptique (31) en forme de plaque, adjacent au corps optique (2), et un deuxième corps haptique (32) en forme de plaque, adjacent au corps optique (2),
**caractérisée en ce que**
le premier corps haptique (31) et le deuxième corps haptique (32) sont agencés avec une symétrie ponctuelle par rapport au centre géométrique (M),
un rayon extérieur (RA) de la lentille intraoculaire (1) autour du centre géométrique M et une distance radiale (A1) du centre géométrique (M) à un point d'intersection (S1) de l'axe transversal (Q) avec une ligne périphérique (12) de la lentille intraoculaire (1) présentant un rapport mutuel qui se situe dans la plage de 1:0,5 à 1:0,9,
au moins deux premiers évidements (4) étant présents dans le premier corps haptique (31), un premier évidement (4) étant présent à gauche de l'axe longitudinal (L) et l'autre premier évidement (4) étant présent à droite de l'axe longitudinal (L), les deux premiers évidements (4) présentant une première longueur d'évidement (L4) et une première largeur d'évidement (B4) respectives, la première longueur d'évidement (L4) étant plus grande que la première largeur d'évidement (B4),
les premiers évidements (4) présentant chacun un centroïde (S4), ces centroïdes respectifs (S4) pouvant être reliés par une ligne qui forme un premier arc de cercle (7) ayant un premier rayon (R1) autour d'un premier centre (M1) agencé sur l'axe longitudinal (L), au moins deux deuxièmes évidements (5) étant présents dans le premier corps haptique (31), un deuxième évidement (5) étant présent à gauche de l'axe longitudinal (L) et l'autre deuxième évidement (5) étant présent à droite de l'axe longitudinal (L), les deux deuxièmes évidements (5) présentant une deuxième longueur d'évidement (L5) et une deuxième largeur d'évidement (B5) respectives, la deuxième longueur d'évidement (L5) étant plus grande que la deuxième largeur d'évidement (B5), les deuxièmes évidements (5) présentant chacun un centroïde (S5), ces centroïdes respectifs (S5) pouvant être reliés par une ligne qui forme un deuxième arc de cercle (8) ayant un deuxième rayon (R2) autour du premier centre (M1), le deuxième rayon (R2) étant plus petit que premier rayon (R1).

2. Lentille intraoculaire (1) selon la revendication 1, dans laquelle le premier évidement (4) ou le deuxième évidement (5) est configuré en forme de fente.

3. Lentille intraoculaire (1) selon l'une quelconque des revendications précédentes, dans laquelle la lentille intraoculaire (1) est formée d'un seul matériau.

4. Lentille intraoculaire (1) selon l'une quelconque des revendications précédentes, dans laquelle le premier évidement (4) ou le deuxième évidement (5) est présent uniquement à l'intérieur du premier corps haptique (31) et n'atteint pas un bord extérieur (10) du corps haptique (3) .

5. Lentille intraoculaire (1) selon l'une quelconque des revendications précédentes, dans laquelle le premier évidement (4) présente une première largeur d'évidement (B4) dans la plage de 0,05 mm à 0,8 mm et/ou le deuxième évidement (5) présente une deuxième largeur d'évidement (B5) dans la plage de 0,05 mm à 0,8 mm.

6. Lentille intraoculaire (1) selon l'une quelconque des revendications précédentes, dans laquelle la lentille intraoculaire (1) présente une face supérieure (20) et une face inférieure (21) et le premier évidement (4) ou le deuxième évidement (5) s'étend en section transversale longitudinale de la face supérieure (20) de la lentille intraoculaire (1) à la face inférieure (21) de la lentille intraoculaire (1).

7. Lentille intraoculaire (1) selon la revendication 6, dans laquelle le premier évidement (4) ou le deuxième évidement (5) présente, en section transversale longitudinale, une largeur (B4) plus grande sur la face supérieure (20) de la lentille intraoculaire (1) que sur la face inférieure (21) de la lentille intraoculaire (1).
